# EUROPEAN PATENT APPLICATION

(11) **EP 4 725 474 A1**
(43) Date of publication of application: **15.04.2026**
(21) Application number: 24300009.8
(22) Date of filing: 11.10.2024
(51) Int. Cl.: A61K 9/00, A61K 9/14, A61K 9/16, A61K 9/20, A61K 31/4025

(54) **A SOLID COMPOSITION OF ELIGLUSTAT**

(71) Applicant: Genepharm S.A., 15351 Pallini (GR)
(72) Inventor: XENOGIORGIS, Vasileios, 17676 Athens (GR); MPENEKIS, Vasilis, 11475 Athens (GR); BEKYROU, Chrysanthi, 15121 Pefki (GR)
(74) Representative: Roukounas, Dimitrios

(57) **Abstract**

A solid pharmaceutical composition comprising a crystalline form of eliglustat free base and a pharmaceutically acceptable excipient, wherein the composition is free of binder.

## Description

### FIELD OF THE INVENTION

The present invention relates to a solid composition of crystalline form R1 of eliglustat free base wherein the composition is free of binder and a process for the preparation of such solid compositions.

### BACKGROUND OF THE INVENTION

Eliglustat is chemically known as N-[(1R,2R)-2-(2,3-dihydro-1,4-benzodioxin-6-yl)-2-hydroxy -1-(1-pyrrolidinyl-methyl)ethyl]-octanamide.

Eliglustat hemitartrate (Genz-112638), is a glucocerebroside (glucosylceramide) synthase inhibitor for the treatment of Gaucher disease and other lysosomal storage disorders. Eliglustat hemitartrate is orally active with potent effects on the primary identified molecular target for type 1 Gaucher disease and other glycosphingolipidoses. Gaucher disease belongs to the class of lysosomal diseases known as glycosphingolipidoses, which result directly or indirectly from the accumulation of glycosphingolipids, many hundreds of which are derived from glucocerebroside. Eliglustat hemitartrate is an improved inhibitor of glucocerebroside synthase.

European Patent EP1409467B1 discloses the compound eliglustat and its preparation method. However, the '467 patent does not suggest any compositions of eliglustat free base or its pharmaceutically acceptable salts.

Various publications provide different salts of eliglustat like European Patent 2504332 prepares the tartrate salt of eliglustat and exemplifies compositions with the hemitartrate salt in capsule form; PCT publication 2015059679 discloses the crystalline form R1 of eliglustat; European Patent 3283483 discloses the hydrochloride salt of eliglustat; PCT publication 2023083293 discloses the oxalate salt of eliglustat crystal form A, naphthalene, disulfonate, mucate and glutarate.

However, none of the publications teach compositions of free base. Further, due to the hygroscopic nature of the salts the composition comprises binder for preparation of the granules for filling in capsules.

It is therefore advantageous to provide compositions of eliglustat free base which would avoid an unnecessary step of preparation of the salt form of eliglustat and the cumbersome work of storing the salt form.

### SUMMARY OF THE INVENTION

The present invention is defined in the appended claims.

In accordance with a first aspect, there is provided a solid composition of crystalline form of eliglustat free base.

The preferred crystalline form of eliglustat free base is form R1.

The solid composition of crystalline form of eliglustat free base is free of binder.

In accordance with a second aspect, there is provided a process for the preparation of a solid composition according to the first aspect.

In embodiments, the solid composition is prepared by using wet granulation.

Certain embodiments of the present invention may provide one or more of the following advantages:
- desired stability of crystalline form of eliglustat free base compositions;
- desired cost of crystalline form of eliglustat free base compositions;
- desired ease of handling of crystalline form of eliglustat free base;
- desired ease of preparation of crystalline form of eliglustat free base compositions

The inventors surprisingly found that a solid composition according to the present invention may exhibit favourable physical attributes sufficient for manufacturing, alongside sufficient stability when compared to a current commercial product. Examples of the present invention were shown to display comparable dissolution and/or assay performance and/or stability when compared to solid compositions without binders.

### BRIEF DESCRIPTION OF THE DRAWINGS

The invention will further be illustrated by reference to the following figures:

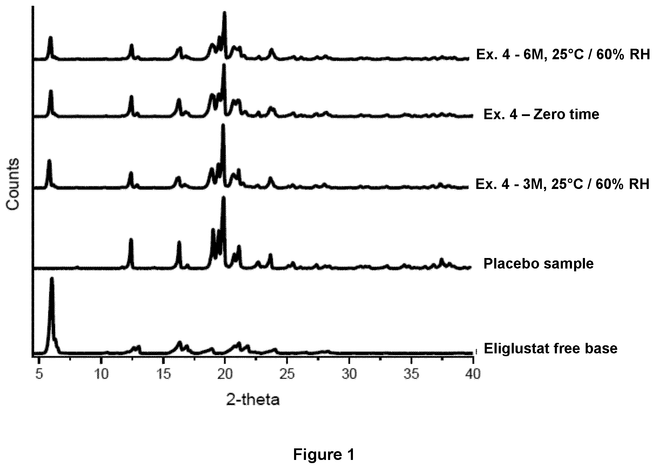

Figure 1: XRD spectrum illustrating the stability of crystalline form R1 of eliglustat free base in solid composition of the present invention.

### DETAILED DESCRIPTION OF THE INVENTION

The present invention is based on the surprising finding that compositions of crystalline eliglustat with good stability can be obtained in the absence of a binder.

Preferably, the crystalline form of eliglustat according to the present invention is crystalline form R1.

When ranges are used herein, all combinations and sub-combinations of ranges and specific embodiments therein are intended to be included.

As used herein, the term "about" when referring to a number or a numerical range means that the number or numerical range referred to is an approximation within experimental variability (or within statistical experimental error), and thus the number or numerical range may vary.

As used herein, the term "comprising" (and related terms such as "comprise" or "comprises" or "having" or "including") has an open meaning and therefore a pharmaceutical composition comprising described features may comprise additional components in addition to the described features.

Abbreviations used herein have their conventional meaning within the chemical and biological arts, unless otherwise indicated.

As used herein, the term "dispersion" generally refers to a system where a substance is dispersed within another substance.

As used herein, the term "solid dispersion" generally refers to the solid-state dispersion of one or more active substances in an inert carrier excipient, for example, one or more poorly soluble drugs within a water-soluble polymer.

As used herein, the term "excipient" refers to an inactive ingredient in a composition.

As used herein, the term "diluent" or "filler" refers to an excipient that adds bulk to a composition.

As used herein, the term "disintegrant" refers to an excipient that promotes disintegration of a tablet / capsule composition into smaller fragments in an aqueous environment.

As used herein, the term "surfactant" refers to an excipient that decreases surface or interfacial tension to impart compositions with enhanced solubility and/or wettability.

As used herein, the term "binder" refers to an excipient that improves cohesion, and plasticity of a composition.

As used herein, the term "treatment" refers to an approach for obtaining beneficial or desired results including, but not limited to, therapeutic benefit and/or a prophylactic benefit. The term "patient" refers to an animal, such as a mammal, for example a human.

As used herein, the term "intragranular" refers to a component added before a granulation process.

As used herein, the term "extragranular" refers to a component added after granulation and before a compression process.

In certain embodiments, a solid composition of crystalline R1 form of eliglustat further comprises a filler/diluent, a disintegrant, a glidant, a lubricant and/or a surfactant,.

In certain embodiments, the solid composition of crystalline R1 form of eliglustat comprises a surfactant selected from sodium lauryl sulfate, poly(ethylene oxide)-poly(propylene oxide)-poly(ethylene oxide), poloxamer, polysorbate 20 and polysorbate 80, or a combination thereof. In some embodiments, the solid composition comprises surfactant in an amount of about 0.1 wt.% to about 5 wt.%, for example, of from about 0.1 wt.% to about 4 wt.%, of from about 0.5 wt.% to about 3 wt.%, or of from about 1 wt.% to about 2 wt.%. In some embodiments, for example, the solid composition according to the present invention comprises a surfactant in an amount of about 2 wt.%.

In certain embodiments, the solid composition is free from intragranular lubricant. The term "free from intragranular lubricant" as used herein refers to compositions where lubricant has not been added to a composition to achieve a particular effect prior to granulation. For example, "free from intragranular lubricant" means that an intragranular lubricant is present in the composition in an amount of less than about 0.5 wt.%, or less than about 0.3 wt.%, or less than about 0.2 wt.%, or less than about 0.1 wt.%, or less than about 0.05 wt.%, or less than about 0.01 wt.%, or less than about 0.005 wt.% based on the total weight of the solid composition.

In certain embodiments, the solid composition of crystalline R1 form of eliglustat free base comprises about 20 wt.% to about 45 wt.% of eliglustat free base; for example, from about 25 wt.% to about 45 wt.% of eliglustat free base.

In certain embodiments, the solid composition of crystalline R1 form of eliglustat comprises from about 20 wt.% to about 45 wt.% of eliglustat free base based on the total weight of the solid composition.

In certain embodiments, the solid composition of crystalline R1 form of eliglustat comprises a filler or diluent selected from silicified microcrystalline cellulose, microcrystalline cellulose, mannitol, starch, pregelatinized starch and lactose, or a combination thereof. In some embodiments, the lactose may be lactose anhydrous. In alternative embodiments, the lactose may be lactose monohydrate. In some embodiments, the solid composition comprises filler or diluent in an amount of about 45 wt.% to about 75 wt.%, for example, from about 45 wt.% to about 70 wt.%. In certain embodiments, the solid composition comprises filler or diluent in an amount of about 50 wt.% to about 70 wt.% based on the total weight of the solid composition.

In certain embodiments, the solid composition of crystalline R1 form of eliglustat comprises a disintegrant selected from pregelatinized starch, croscarmellose sodium and crospovidone, or a combination thereof. In some embodiments, the solid composition comprises disintegrant in an amount of about 1 wt.% to about 10 wt.%, for example, from about 1 wt.% to about 9 wt.%, from about 2 wt.% to about 8 wt.%, from about 3 wt.% to about 7 wt.%, or from about 4 wt.% to about 6 wt.%. In certain embodiments, the solid composition comprises disintegrant in an amount from about 5 wt.% to about 8 wt.% based on the total weight of the solid composition.

In certain embodiments, the solid composition of crystalline R1 form of eliglustat comprises a glidant selected from colloidal silica, fumed silica, talc and magnesium carbonate, or a combination thereof. In some embodiments, the solid composition comprises glidant in an amount of about 0.1 wt.% to about 5 wt.%, for example, from about 0.5 wt.% to about 4.5 wt.%, from about 1 wt.% to about 4 wt.%, or from about 1 wt.% to about 3 wt.%. In some embodiments, for example, the solid composition according to the present invention comprises a glidant in an amount of about 2 wt.% based on the total weight of the solid composition.

In certain embodiments, the solid composition of crystalline R1 form of eliglustat comprises a lubricant selected from magnesium stearate, sodium stearyl fumarate, glyceryl behenate (such as Compritol 888^{®}) or a combination thereof. In some embodiments, the solid composition comprises lubricant in an amount of from about 0.1 wt.% to about 5 wt.%, for example, from about 0.5 wt.% to about 4.5 wt.%, from about 1 wt.% to about 4 wt.%, or from about 2 wt.% to about 4 wt.%. In some embodiments, for example, the solid composition according to the present invention comprises a lubricant in an amount of about 3 wt.% based on the total weight of the solid composition.

Binders are substances that are added either dry or in liquid form during wet granulation to form granules or to promote cohesive compacts for directly compressed tablets. E.g., starch, pregelatinized starch, PEG, sorbitol, and HPMC, etc. Binders and disintegrants in general, have opposite uses in an oral solid formulation. Binder delays disintegration while disintegrant increase disintegration. In order to prevent a delayed disintegration time and as a result maybe an impact on dissolution rate versus innovator, the inventors eliminated the presence of a binder agent.

In certain embodiments, the solid composition of crystalline R1 form of eliglustat is free from solid dispersions. Solid dispersions may be prepared by mixing eliglustat with a polymer and subjecting the mixture to spray drying or hot melt extrusion process. Hot-melt extrusion is a manufacturing process where polymeric materials are heated above their glass-transition temperature and mechanically mixed with active compounds in order to disperse the drug substance molecularly into the polymeric net. Spray -drying consists in two main steps: i) complete dissolution of the drug substance and a dilution material (e.g., a polymer) in a solvent or mixture of solvents and ii) fast spray -drying of the previous solution in order to collect the solid material. The polymer may be selected from HPMC, HPMCAS, PVP, HPC, methacrylic acid/methacrylate copolymers or any combinations thereof.

In certain embodiments, the solid composition of crystalline R1 form of eliglustat is a free-flowing powder, granules, capsules, tablets, films, sublingual tablets or mouth dissolving tablets and pellets.

Preferred embodiment of the solid composition of crystalline form of eliglustat free base of the present invention is capsules.

In certain embodiments, the solid composition of crystalline R1 form of eliglustat free base comprises:
(a) crystalline R1 form of eliglustat free base in an amount from about 20 wt.% to about 45 wt.%;
(b) a surfactant in an amount from about 0.1 wt.% to about 5 wt.%;
(c) a diluent or a filler in an amount from about 45 wt.% to about 70 wt.%;
(d) a disintegrant in an amount from about 1 wt.% to about 10 by wt.%;
(e) a glidant in an amount from about 0.1 wt.% to about 5 wt.%;
(f) a lubricant in an amount from about 0.1 wt.% to about 10 wt.%;
wherein each component is based on the total weight of the solid composition.

In certain embodiments, the solid composition of crystalline R1 form of eliglustat free base comprises:
(a) crystalline R1 form of eliglustat free base in an amount from about 20 wt.% to about 45 wt.%;
(b) a filler or a diluent in an amount from about 45 wt.% to about 75 wt.%;
(c) a disintegrant in an amount from about 4 wt.% to about 8 wt.%;
(d) a glidant in an amount from about 0.1 wt.% to about 4 wt.%;
(e) a lubricant in an amount from about 0.1 wt.% to about 4 wt.%;
wherein each component is based on the total weight of the solid composition.

In certain embodiments, the solid composition of crystalline R1 form of eliglustat free base comprises:
(a) crystalline R1 form of eliglustat in an amount from about 20 wt.% to about 45 wt.%;
(b) a filler or a diluent in an amount from about 45 wt.% to about 75 wt.%;
(c) a glidant in an amount from about 0.1 wt.% to about 4 wt.%;
(d) a lubricant in an amount from about 0.1 wt.% to about 10 wt.%;
wherein each component is based on the total weight of the solid composition.

In certain embodiments, the solid composition of crystalline R1 form of eliglustat free base comprises:
(a) crystalline R1 form of eliglustat in an amount from about 20 wt.% to about 45 wt.%;
(b) a filler or a diluent in an amount from about 45 wt.% to about 75 wt.%;
(c) a lubricant in an amount from about 1 wt.% to about 10 wt.%;
wherein each component is based on the total weight of the solid composition.

In certain embodiments, the solid composition of crystalline R1 form of eliglustat free base comprises:
(a) crystalline form R1 of eliglustat free base in an amount from about 20 wt.% to about 45 wt.%;
(b) a diluent or a filler in an amount from about 45 wt.% to about 75 wt.%;
(c) a lubricant in an amount from about 4 wt.% to about 12 wt.%;
wherein each component is based on the total weight of the solid composition and the diluent or filler comprises lactose and microcrystalline cellulose in ratio of 8:1 to 12:1 by weight and the solid composition is capsules.

In certain embodiments, the solid composition of crystalline R1 form of eliglustat is coated with a polymeric film coating material. Numerous suitable polymeric film coating materials are known in the art. In certain embodiments, the polymeric film coating material is selected from a film-coating polymer such as polyvinyl alcohol and/or polyethylene glycol. In some embodiments, the polymeric film-coating polymer is selected from hydroxypropylmethylcellulose or hydroxypropylcellulose.

In certain embodiments, the solid composition of crystalline R1 form of eliglustat free base is an immediate release composition.

As used herein, the term "immediate release" generally refers to a composition that is developed to dissolve without delaying or prolonging dissolution or absorption of the drug.

In certain embodiments, the dissolution of eliglustat from a capsule formed from the solid composition of the present invention is at least about 75 % when the capsule is subjected to dissolution tests at pH range from 1.2 to 6.8 at 37° C ± 0.5°C using a USPII paddle apparatus with stirring speed of 50 rpm for 30 minutes. For example, the dissolution of eliglustat from a capsule formed from the solid composition of the present invention is at least about 80%, at least about 85%, or at least about 90% when the capsule is subjected to dissolution tests at a pH range from 1.2 to 6.8 at 37° C ± 0.5°C using a USPII paddle apparatus with stirring speed of 50 rpm for 30 minutes. Such dissolution is beneficial, as it shows the capsule has good bioavailability. A capsule formed from the solid composition of the present invention having a high percentage of dissolution may illustrate the rate at which the active ingredient would be released during treatment of a patient.

In certain embodiments, the solid composition of crystalline R1 form of eliglustat free base is stable for at least about 1 month, at least about 2 months, at least about 3 months, at least about 4 months, at least about 5 months, or at least about 6 months at about 40°C and about 75% relative humidity & at about 25 °C and about 60% relative humidity. The term stable is defined herein with regards to polymorphic stability and/or chemical stability. The chemical stability may be measured by suitable methods such as X-ray diffraction (XRD), dissolution and High-Performance Liquid Chromatography (HPLC). For example, the crystalline nature and active purity of the solid composition of crystalline eliglustat is retained at about 40°C and about 75% relative humidity & at about 25 °C and about 60% relative humidity for at least about 3 months. For example, the crystalline nature and active purity of the solid composition of crystalline eliglustat is retained at about 40 °C and about 75% relative humidity & at about 25 °C and about 60% relative humidity for at least about 6 months.

In certain embodiments, the capsules formed from the solid composition of the present invention comprise up to about 84 mg of eliglustat in a single dose. The capsule compositions of eliglustat are suitable for oral administration of eliglustat as a treatment for a patient.

In certain embodiments the solid composition of crystalline R1 form of eliglustat may be prepared by wet granulation or dry granulation followed by filling in capsules. The preferred process is wet granulation using purified water.

In certain embodiments, the solid composition may have one or more of the following effects:
- increased simplicity of the composition;
- increased stability of the composition;
- maintenance of the stability of the composition;
- reduced cost of the composition;
- increased ease of preparing the composition.

### EXAMPLES

### Test methods and methodology

XRD was performed according to the methodology of Chapter 2.9.33 of the European Pharmacopoeia, 11th Edition (2023), using a Bruker D2 Phaser Instrument, with a Cu Kα (λ=1.54 Å) source and a LynxEye detector.

The d₉₀ particle size of the crystalline R1 form of eliglustat is measured in a well-known manner by a laser light diffraction according to the method of Chapter 2.9.31 of the European Pharmacopoeia, 11th Edition (2023). A Malvern Mastersizer (3000), Hydro-3000MV (accessory) and Analytical balance were used for d₉₀ particle size determination. Particle size is determined by passing a laser beam through a dispersed sample and measuring the variation in angular scattered light intensity. The median particle size d₉₀ is the value at which there are 90% by number of particles which have an equivalent spherical diameter less than that d₉₀ value.

Dissolution tests at a pH range from 1.2 to 6.8 at 37° C ± 0.5°C using a USPII paddle apparatus with stirring speed of 50 rpm and 900ml volume for 30 minutes.

The following illustrates examples of the solid composition of crystalline form of eliglustat, and related aspects described herein. Thus, these examples should not be considered to restrict the present disclosure but are merely in place to teach how to carry out the present disclosure.

### Section I: Solid compositions of the present invention

| EXAMPLES | 1 | 2 | 3 | 4 |
|---|---|---|---|---|
| | mg/cap | mg/cap | mg/cap | mg/cap |
| Intragranular excipients | | | | |
| Eliglustat free base | 84.40 | 84.40 | 84.40 | 84.40 |
| MCC 101 | 42.50 | 43.82 | 27.93 | 16.20 |
| Tablettose 70 | 135.00 | 136.35 | 125.27 | 150.50 |
| Methocel E15 | 5.40 | 5.40 | 5.40 | - |
| Methocel E5 | - | - | - | - |

| Wet granulation | | | | |
|---|---|---|---|---|
| Purified water | q.s. | q.s. | q.s. | 62.54 |

| Lubrication | | | | |
|---|---|---|---|---|
| Glyceryl behenate (compritol 888) | 2.70 | - | - | 18.9 |
| Starch 1500 | - | - | 27.00 | - |
| Capsule filling weight | 270.00 | 270.00 | 270.0 | 270.00 |
| Caps No.1 pearl white pearl green op. prin | | | | |

### Section II: Dissolution study in 0.1N HCI with 50 rpm

| **BATCH** | **RLD** | **Example 4** |
|---|---|---|
| DISS. MED | HCL 0.1 N | HCL 0.1 N |
| APPARATUS | PADDLES | PADDLES |
| RPM | 50 | 50 |
| VOLUME | 900 | 900 |
| INSTRUMENT | DISTEK | DISTEK |
| 5 MIN | 55.0 | 73.8 |
| 10 MIN | 80.5 | 97.4 |
| 15 MIN | 90.8 | 99.0 |
| 30 MIN | 99.8 | 100.9 |

In the table above, RLD refers to pharmaceutical product Cerdelga^{®} 84mg capsules, which has the qualitative composition shown in the table below.

| **CERDELGA^{®} 84mg Capsules** | |
|---|---|
| Capsule contents | |
| | Microcrystalline cellulose |
| | Lactose monohydrate |
| | Hypromellose |
| | Glycerol dibehenate |

| Capsule shell | |
|---|---|
| | Gelatin |
| | Potassium aluminium silicate (E555) |
| | Titanium dioxide (E171) |
| | Yellow iron oxide (E172) |
| | Indigotine (E132) |

| Printing ink | |
|---|---|
| | Shellac |
| | Black iron oxide (E172) |
| | Propylene glycol |
| | Ammonia solution, concentrated |

### Section III: Stability studies

The polymorphic stability of Example 4 was also investigated using XRD. The stability of Example 4 after 3 months and after 6 months of being stored in high-density polyethylene, with desiccant, and stored at 25 °C and 60 % relative humidity was investigated. A placebo sample was prepared with the same qualitative and quantitative (adjusted to 100% final ratio) composition as per Example 4, without the use of the eliglustat active ingredient. As shown in Figure 1, after both 3 months and 6 months Example 4 maintains its crystallinity and shows good polymorphic stability. Figure 4 also demonstrates the crystalline nature of the eliglustat active ingredient used.

## Claims

1. A solid pharmaceutical composition comprising a crystalline form of eliglustat free base and a pharmaceutically acceptable excipient, wherein the composition is free of binder.

2. The solid pharmaceutical composition according to claim 1, wherein the crystalline form is form R1.

3. The solid pharmaceutical composition according to any one of the preceding claims, wherein the composition is prepared by wet granulation.

4. The solid pharmaceutical composition according to any one of the preceding claims, wherein the pharmaceutically acceptable excipient is selected from diluent or filler, disintegrant, lubricant, glidant, stabilizer and surfactant or mixture thereof.

5. The solid pharmaceutical composition according to any one of the preceding claims, wherein the composition is a solid dosage form selected from powder, granules, capsules, tablets, films, sublingual tablets or mouth dissolving tablets and pellets.

6. The solid pharmaceutical composition according to any one of the preceding claims, wherein the composition comprises:
(a) crystalline form R1 of eliglustat free base in an amount from about 20 wt.% to about 45 wt.%;
(b) a diluent or a filler in an amount from about 45 wt.% to about 75 wt.%;
(c) a lubricant in an amount from about 4 wt.% to about 12 wt.%;
wherein each component is based on the total weight of the solid composition.

7. The solid pharmaceutical composition according to any one of the preceding claims, wherein the composition comprises
(a) crystalline form R1 of eliglustat free base in an amount from about 20 wt.% to about 45 wt.%;
(b) a diluent or a filler in an amount from about 45 wt.% to about 75 wt.%;
(c) a lubricant in an amount from about 4 wt.% to about 12 wt.%;
wherein each component is based on the total weight of the solid composition and the diluent or filler comprises lactose and microcrystalline cellulose in ratio of 8:1 to 12:1 by weight and the solid composition is capsules.

8. The solid pharmaceutical composition according to any one of the preceding claims, wherein the crystalline form R1 of eliglustat is retained for at least 12 months at 40 °C and 75% relative humidity.

9. The solid pharmaceutical composition according to any one of the preceding claims, wherein the composition is an immediate release composition.

10. A solid pharmaceutical composition comprising
(a) crystalline form R1 of eliglustat free base in an amount from about 20 wt.% to about 45 wt.%;
(b) microcrystalline cellulose in an amount from about 4 wt.% to about 10 wt.%;
(c) lactose or lactose monohydrate in an amount from about 45 wt.% to about 65 wt.%; and
(d) glyceryl behenate in an amount from about 5 wt.% to about 10 wt.%; wherein each component is based on the total weight of the solid composition.

11. The solid pharmaceutical composition according to claim 10, wherein the composition is in capsule form.

12. The solid pharmaceutical composition according to claim 10 or 11, wherein the crystalline form R1 of eliglustat is retained for at least 12 months at 40 °C and 75% relative humidity.

13. The solid pharmaceutical composition according to any one of claims 10 to 12 wherein the composition is an immediate release composition.
